# EUROPÄISCHE PATENTSCHRIFT

(11) **EP 0 936 206 B1**
(45) Veröffentlichungstag und Bekanntmachung des Hinweises auf die Patenterteilung: **09.10.2002**
(21) Anmeldenummer: 99101727.8
(22) Anmeldetag: 10.02.1999
(51) Int. Cl.: C07C 6/04, C07C 11/06, C07C 11/08

(54) **Verfahren zur Herstellung von Propen und gegebenenfalls 1-Buten**
Process for preparing proylene and 1-butene
Procédé de préparation de propylène ainsi que 1-butène

(30) Priorität: 12.02.1998 DE 19805716
(43) Veröffentlichungstag der Anmeldung: 18.08.1999
(73) Patentinhaber: BASF AKTIENGESELLSCHAFT, 67056 Ludwigshafen (DE)
(72) Erfinder: Schwab, Peter, Dr., 67098 Bad Dürkheim (DE); Breitscheidel, Boris, Dr., 67117 Limburgerhof (DE); Oost, Carsten, Dr., 67098 Bad Dürkheim (DE); Schulz, Ralf, Dr., 67346 Speyer (DE); Schulz, Michael, Dr., 67067 Ludwigshafen (DE)
(74) Vertreter: Isenbruck, Günter, Dr.

(56) Entgegenhaltungen:
- EP-A- 0 304 515
- EP-A- 0 832 867
- US-A- 5 120 894

## Beschreibung

Die Erfindung betrifft ein Verfahren zur Herstellung von Propen und gegebenenfalls 1-Buten durch Metathese von Olefinen.

Die Olefinmetathese (Disproportionierung) beschreibt in ihrer einfachsten Form die reversible, metallkatalysierte Umalkylidenierung von Olefinen durch Bruch und Neuformierung von C=C-Doppelbindungen. Dabei werden beispielsweise Olefine der Formeln R¹-CH=CH-R² und R³-CH=CH-R⁴ reversibel zu Olefinen der Formeln R¹-CH=CH-R³ und R²-CH=CH-R⁴ umgesetzt. Bei der Metathese acyclischer Olefine unterscheidet man zwischen Selbstmetathese, bei der ein Olefin in ein Gemisch zweier Olefine unterschiedlicher molarer Massen übergeht, und Kreuz- oder Co-Metathese, bei der zwei unterschiedliche Olefine reagieren. Ein Beispiel für eine Selbstmetathese ist die Umsetzung von zwei Molekülen Propen zu Ethen und 2-Buten, wie sie beispielsweise nach dem Phillips-Triolefin-Verfahren ausgeführt wird, siehe Hydrocarbon Processing. Band 46, Nr. 11, November 1967, S. 232. Ein Beispiel für eine Kreuz-Metathese ist die Umsetzung von Propen und 1-Buten zu Ethen und 2-Penten. Ist einer der Reaktionspartner Ethen, so spricht man üblicherweise von einer Ethenolyse.

Die Metathesereaktionen erfolgen in Gegenwart von Katalysatoren. Hierzu eignen sich prinzipiell homogene und heterogene Übergangsmetallverbindungen, insbesondere solche der VI bis VIII Nebengruppe des Periodensystems der Elemente, wie auch homogene und heterogene Katalysatorsysteme, in denen diese Verbindungen enthalten sind.

Aus DE-A-19 40 433 ist die Metathese von 1-Buten mit 2-Buten zu Propen und 2-Penten bekannt, wobei Re₂O₇/Al₂O₃ als Katalysator verwendet wird. Das gebildete 2-Penten wird mit Natriumhydrid auf Kaliumcarbonat und Ethen zu Heptenen weiter umgesetzt.

Die Methathese von 1-Buten und 2-Buten zu Propen und 2-Penten wird in K.L. Anderson, T.D. Brown, Hydrocarbon Processing, Band 55, Nr.8, August 1978, S.119-122 als Nebenreaktion bei der Synthese von Isoamylen aufgeführt.

Aus EP-A-0 304 515 ist ein Metatheseverfahren zur Umsetzung von 1-Buten mit 2-Buten zu Propen und Pentenen bekannt, das in einer Reaktivdestillationsvorrichtung mit Re₂O₇/Al₂O₃ als Katalysator ausgeführt wird.

Aus US 3,526,676 ist die Metathese von 1-Buten mit 2-Buten zu Propen und Penten bekannt. Sie wird an MoO₃ und CoO auf Al₂O₃ durchgeführt

Aus US 3,785,957 ist ein Verfahren zur Herstellung von Treibstoff mit hoher Octanzahl bekannt. Dabei wird ein olefinischer Treibstoff mit Ethylen disproportioniert und der Austrag aufgetrennt in einen Propylenstrom, einen Butenstrom, einen C₅oder C₅₋₆-Olefinstrom und einen C₆₊- oder C₇₊-Treibstoffstrom. Der C₅- oder C₅₋₆-Olefinstrom wird mit Ethen an einem WO₃/SiO₂-Festbettkatalysator disproportioniert, um Propylen und Butene zu erhalten. Das erhaltene Propylen wird disproportioniert zu Ethylen und Butenen und die Butene werden mit Isobutan alkyliert.

Aus US 3,767,565 ist ein Verfahren zur Erhöhung der Octanzahl von Treibstoff bekannt, wobei ein C₅-Schnitt eines olefinischen Treibstoff mit Ethylen in Gegenwart eines Katalysators aus WO₃/SiO₂ und MgO umgesetzt wird zu Ethylen, Propylen, n-Butenen und Isobutenen. Das erhaltene Propylen wird disproportioniert und die erhaltenen n-Butene werden mit Isobutan alkyliert.

Aus EP-A-0 691 318 ist ein Olefinmetatheseverfahren bekannt, bei dem C₅-Olefine und Ethylen in Gegenwart eines Katalysators zu gemischten C₄-Olefinen und Propen umgesetzt werden. So wird 2-Methyl-2-buten mit Ethen zu Isobuten und Propen umgesetzt. Ein Gemisch aus 2-Pentenen und 2-Methyl-2-buten wird zu einem Gemisch aus 1-Buten, Isobuten und Propen umgesetzt.

In den vorstehenden Verfahren erfolgt die Herstellung von Propen unter Zudosierung mindestens äquimolarer Mengen an Ethen. Zur Erzielung hoher Propenselektivitäten muß Ethen in großer Menge im Kreis gefahren werden.

Aufgabe der vorliegenden Erfindung ist die Bereitstellung eines Verfahrens zur Herstellung von Propen und gegebenenfalls 1-Buten als Koppelprodukt in variablen Mengen unter Verwendung von C₄-Olefinen wie Raffinat II.

Die Aufgaben werden erfindungsgemäß gelöst durch ein Verfahren zur Herstellung von Propen und gegebenenfalls 1-Buten durch
a) Umsetzung von 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) zumindest teilweises Ausschleusen des gebildeten 1-Butens und/oder zumindest teilweises Isomerisieren des gebildeten 1-Butens zu 2-Buten in Gegenwart eines Isomerisierungskatalysators und anschließendes Rückführen des nicht ausgeschleusten 1-Butens und gebildeten 2-Butens gemeinsam mit einem Teil der in Schritt a) nicht umgesetzten C₄-Fraktion in Schritt a).

Das erfindungsgemäße Verfahren beinhaltet 2 Metatheseschritte. Im ersten Schritt werden 1-Buten und 2-Buten zu Propen und 2-Penten umgesetzt. In einem zweiten Schritt wird dann 2-Penten mit Ethen zu 1-Buten und Propen umgesetzt. Das 1-Buten wird gemäß einer Ausführungsform der Erfindung dabei zumindest teilweise in Gegenwart eines Isomerisierungskatalysators zu 2-Buten isomerisiert und das resultierende Gemisch aus 1-Buten und 2-Buten in den ersten Reaktionsschritt zurückgeführt. Dabei kann ein Teil des 1-Butens vor der Isomerisierung ausgeschleust oder am Isomerisierungsreaktor vorbeigeleitet und mit dem Austrag des Isomerisierungsreaktors vereinigt werden. Hierdurch wie auch durch Regelung des Umsatzes am Isomerisierungskatalysator kann ein optimales Mengenverhältnis von 1-Buten zu 2-Buten am Eingang des ersten Metathesereaktors eingestellt werden, um eine maximale Ausbeute an Propen zu erreichen. Der Rohstoffbedarf an Ethen und C₄-Olefinen kann so gegenüber einstufigen Ethenolyseverfahren, wie sie beispielsweise in US 3,660,506 und EP-A-0 273 817 beschrieben sind, um etwa 5 bis 15% verringert werden.

Gemäß einer Ausführungsform wird das in Schritt c) gebildete 1-Buten teilweise in Gegenwart eines Isomerisierungskatalysators zu 2-Buten isomerisiert und das resultierende Gemisch aus 1-Buten und 2-Buten in Schritt a) zurückgeführt.

Gemäß einer weiteren Ausführungsform wird das in Schritt c) gebildete 1-Buten zumindest teilweise ausgeschleust und nicht ausgeschleustes 1-Buten in Schritt a) zurückgeführt.

Als Nettoreaktion ergibt sich somit die Umsetzung von 2-Buten mit Ethen zu 2 Molekülen Propen. Bei der Umsetzung von 2-Penten mit Ethen werden gemäß einer Ausführungsform der Erfindung formal nur equimolare Mengen an Einsatzstoffen benötigt, um mit hoher Ausbeute zu den Produkten zu gelangen. Somit kann im Gegensatz zum umgekehrten Trioolefin-Verfahren die eingesetzte Ethenmenge geringgehalten werden.

Beide Metatheseschritte können als Reaktivdestillation ausgeführt werden, wie sie nachstehend beschrieben ist.

1-Buten und 2-Buten können in der Umsetzung als reine Stoffe eingesetzt werden gemäß einer Ausführungsform der Erfindung. Gemäß einer anderen Ausführungsform der Erfindung werden die Butene in Form eine C₄-Stroms eingesetzt, der beispielsweise aus einem Cracker, insbesondere Steamcracker, oder einer Raffination stammt. Der C₄-Strom kann dabei neben den Butenen C₄-Alkane enthalten. Gemäß einer Ausführungsform der Erfindung wird ein C₄-Strom verwendet, der aus Raffinat II besteht. Raffinat II ist dabei eine Fraktion aus 1-Buten, cis/trans-2-Buten, wenig oder keinem Isobuten sowie n-Butan und Isobutan. Beispielsweise kann Raffinat II 80 bis 85 Gew.-% Olefine und 15 bis 20 Gew.-% Butane enthalten, mit beispielsweise 25 bis 50 Gew.-% 1-Buten, 30 bis 55 Gew.-% 2-Buten, maximal 1 bis 2 Gew.-% Isobuten. Gemäß einer Ausführungsform der Erfindung besitzt der eingesetzte C₄-Strom einen Anteil an Butenen von 20 bis 100, vorzugsweise 50 bis 90, insbesondere 70 bis 90 Gew.-%. Das Verhältnis von 1-Buten zu 2-Buten beträgt dabei 10:1 bis 1:10, vorzugsweise 3:1 bis 1:3, insbesondere 1:1. Gemäß einer Ausführungsform der Erfindung kann der C₄-Strom geringe Mengen anderer Kohlenwasserstoffe enthalten.

Gemäß einer Ausführungsform der Erfindung kann als Ausgangsstoff jeder Strom verwendet werden, der 1-Buten und 2-Buten enthält. Gemäß einer Ausführungsform der Erfindung kann dabei das 1-Buten aus der erfindungsgemäßen Synthese selbst stammen und mit zugeführtem 2-Buten gemischt werden.

Der eingesetzte C₄-Feedstrom wird vorzugsweise vor dem Einsatz im erfindungsgemäßen Verfahren vorgereinigt, um gegebenenfalls vorliegende Spuren an Wasser, Oxygenates, Schwefel enthaltenden Verbindungen oder Chloriden zu entfernen. Vorzugsweise erfolgt die Entfernung durch Leiten des C₄-Feedstroms über Absorbermaterialien, wie Aluminiumoxid oder Molsiebe, bevorzugt NaX-Molsieb. Die Absorbermaterialien liegen vorzugsweise als Schutzbett (guard bed) vor.

Neben der Umsetzung von 1-Buten und 2-Buten zu Propen und 2-Penten kann ein geringer Anteil an 3-Hexen und Ethen als Nebenprodukt erhalten werden. Zudem können auch geringe Mengen höhersiedender Verbindungen vorliegen.

Die geringen Mengen an Nebenprodukten, die gemäß einer Ausführungsform der Erfindung 1 bis 30 Gew.-%, vorzugsweise 5 bis 20 Gew.-%, bezogen auf die Mengen an gebildetem 2-Penten ausmachen, stören bei der nachfolgenden Umsetzung nicht, so daß gemäß einer Ausführungsform der Erfindung keine Reinigung des 2-Pentens von diesen Nebenprodukten vor der weiteren Umsetzung erforderlich ist. Gemäß einer Ausführungsform der Erfindung wird das 2-Penten in reiner Form in der zweiten Umsetzung eingesetzt.

Somit werden unter dem Ausdruck "2-Penten" auch solche Gemische verstanden, die neben 2-Penten geringe Mengen an Hexenen, insbesondere 3-Hexen, und anderen höhersiedenden Verbindungen enthalten.

Entsprechend wird unter dem Ausdruck "Butene", wie "1-Buten" und "2-Buten" auch ein Gemisch verstanden, das neben dem Buten oder den Butenen C₄-Alkane, insbesondere Butane enthält.

Mehrere Ausführungsformen der Erfindung werden nachstehend anhand der Zeichnung erläutert, in der
- Figur 1: eine schematische Darstellung einer Ausführungsform des erfindungsgemäßen Verfahrens zeigt.
- Figur 2: eine schematische Darstellung einer weiteren Ausführunsform des erfindungsgemäßen Verfahrens zeigen.

Dabei bedeuten die in den Figuren verwendeten Abkürzungen folgendes:
- 1-Bu:: 1-Buten
- 2-Bu:: 2-Buten
- Bu:: Butane
- Et:: Ethen
- Pr:: Propen
- 2-Pe:: 2-Penten
- 3-He:: 3-Hexen
- H:: Hochsieder
- II:: Raffinat II
- C4:: C4-Olefine
- C5⁺:: Olefine mit 5 oder mehr Kohlenstoffatomen
- R01:: Reaktor (Metathese)
- R02:: Reaktor (Metathese)
- R03:: Reaktor (Isomerisierung)
- K101:: Destillationskolonne (vorzugsweise eine Trennwandkolonne, Seitenkolonne oder 2-Kolonnen-Schaltung)
- K201:: Destillationskolonne (vorzugsweise eine Trennwandkolonne, Seitenkolonne oder 2-Kolonnen-Schaltung)
- K301:: Destillationskolonne

Nachstehend wird eine bevorzugte Ausführungsform des erfindungsgemäßen Verfahrens beschrieben, umfassend
a) Umsetzung von 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) zumindest teilweises Isomerisieren des gebildeten 1-Butens zu 2-Buten in Gegenwart eines Isomerisierungskatalysators und anschließendes Rückführen des 1-Butens und gebildeten 2-Butens gemeinsam mit einem Teil der in Schritt a) nicht umgesetzten C₄-Fraktion in Schritt a).

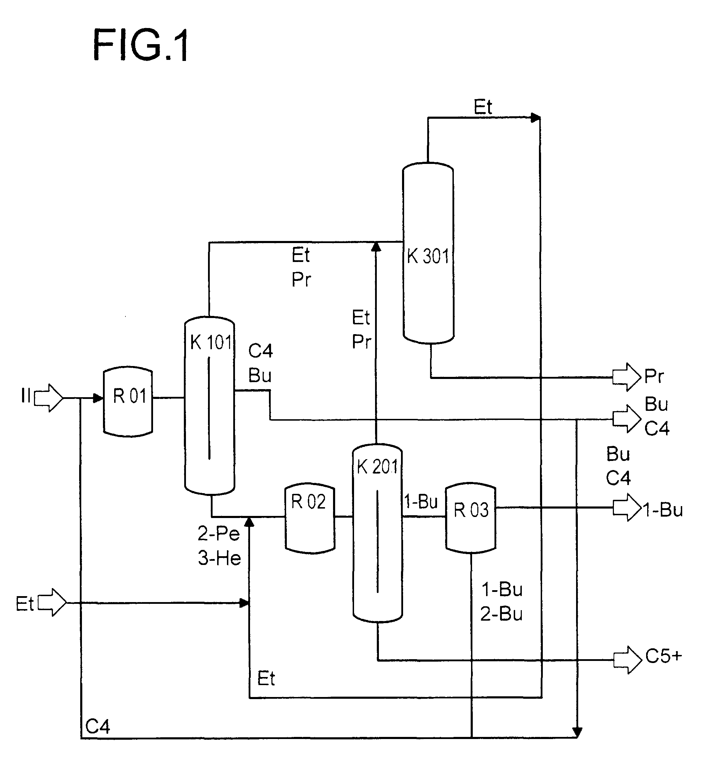

Diese Ausführungsform ist in Fig. 1 dargestellt.

In einem ersten Reaktor R01 werden in Gegenwart des erfindungsgemäßen Metathesekatalysators 1-Buten und 2-Buten zu Propen und 2-Penten umgesetzt. Dazu wird ein Raffinat II-Strom in den Reaktor geführt. An den Reaktor schließt sich eine als Trennwandkolonne, Seitenkolonne oder 2-Kolonnen-Schaltung ausgelegte Destillationskolonne K101 an, an deren Kopf Propen und als Nebenprodukt gebildetes Ethen entfernt werden. Nicht umgesetztes Raffinat II wird im Mittelabzug entfernt und teilweise in den Feedstrom von Raffinat II zurückgeführt. Es kann auch teilweise ausgeschleust werden. Aus dem Sumpf von K101 werden 2-Penten und als Nebenprodukt gebildetes 3-Hexen sowie Hochsieder entnommen. Der Sumpf wird sodann zusammen mit eingespeistem Ethen einem Reaktor R02 zugeführt, der wiederum einen erfindungsgemäßen Metathesekatalysator enthält. In diesem Reaktor R02 findet die Umsetzung von 2-Penten mit Ethen zu 1-Buten und Propen statt. Der Austrag aus Reaktor R02 wird einer als Trennwandkolonne, Seitenkolonne oder 2-Kolonnen-Schaltung ausgelegten Destillationskolonne K201 zugeführt, an deren Kopf Propen und nicht umgesetztes Ethen abgeführt werden. Im Mittelabzug wird gebildetes 1-Buten gegebenenfalls teilweise ausgetragen, und vorzugsweise zumindest teilweise in den Isomerisierungsreaktor R03 geführt. Im Sumpf von K201 fallen nicht umgesetztes 2-Penten, sowie als Nebenprodukte 3-Hexen und Hochsieder an. Diese werden ausgeschleust oder vorzugsweise in R02 zurückgeführt. Die aus K101 und K201 am Kopf abgeführten Gemische aus Propen und Nebenprodukt Ethen werden in einer weiteren Destillationskolonne K301 aufgetrennt. Am Kopf von K301 wird Ethen gewonnen, das vorzugsweise in den Reaktor R02 (zurück)geführt wird. Im Isomerisierungsreaktor R03 wird das 1-Buten an einem Isomerisierungskatalysator zumindest teilweise zu 2-Buten isomerisiert, und das Isomerisierungsgemisch wird in den Reaktor R01 zurückgeführt. Die unterbrochene Linie in R03 deutet die mögliche Ausschleusung des 1-Buten an. Das im Sumpf von K301 erhaltene Propen ist -neben gegebenenfalls ausgeschleusten 1-Buten aus K201 - das gewünschte Umsetzungsprodukt des erfindungsgemäßen Verfahrens. K101 und K201 sind so ausgelegt, daß am Kopf der Kolonne eine leichtsiedende Phase, insbesondere eine C_{2/3}-Phase, die Ethen und Propen enthält, abgeführt wird. Als Mittelsiederphase werden C₄-Ströme abgeführt, insbesondere Butene und Butane. In der Sumpfphase werden C_{≥5}-Kohlenwasserstoffe ausgetragen.

Zwischen den Schritten b) und c) kann der abgetrennte, 2-Penten und 3-Hexen enthaltende Hochsiederaustrag einer Destillation zur Trennung von 2-Penten und 3-Hexen unterworfen werden. Die Destillation kann in jeder geeigneten Apparatur durchgeführt werden. Die 2-Penten enthaltende Fraktion wird danach dem Reaktor R02 zugeführt. Das 3-Hexen kann ausgeschleust und beispielsweise einer Dimerisierung zu einem C₁₂-Olefingemisch zugeführt werden.

Die Reaktoren R01, R02 und R03 können beliebige geeignete Reaktoren sein. Sie können zur kontinuierlichen oder diskontinuierlichen Reaktionsführung dienen. Somit können Sie gemäß einer Ausführungsform Druckgefäße, wie Druckglasgefäße, gemäß einer weiteren Ausführungsform Rohrreaktoren oder Reaktionskolonnen sein. Bevorzugt sind Rohrreaktoren.

Gemäß einer Ausführungsform der Erfindung beträgt der Gesamtumsatz in R01 20 bis 90, vorzugsweise 50 bis 80%.

Gemäß einer Ausführungsform der Erfindung beträgt der Gesamtumsatz in R02 20 bis 100, vorzugsweise 60 bis 90%.

Vorzugsweise findet die Umsetzung in R01 in flüssiger Phase statt. Dabei werden Druck und Temperatur so gewählt, daß die Reaktionspartner in der flüssigen Phase verbleiben.

Gemäß einer Ausführungsform der Erfindung beträgt die Temperatur in R01 0 bis 150°C, vorzugsweise 20 bis 80°C. Der Druck beträgt gemäß einer Ausführungsform der Erfindung 1 bis 200 bar, vorzugsweise 5 bis 30 bar. Die Umsetzung in R02 (Ethenolyse) wird gemäß einer Ausführungsform der Erfindung bei Temperaturen von 0 bis 150°C, vorzugsweise 20 bis 80°C unter einem Ethendruck von 1 bis 200 bar, vorzugsweise 20 bis 80 bar durchgeführt Ethen kann dabei kontinuierlich nachgepreßt werden, so daß ein konstanter Druck eingehalten wird.

Die Umsetzungen in R01 und R02 können für eine Zeit von einer Sekunde bis 10 Stunden, vorzugsweise 1 bis 60 Minuten erfolgen.

Bei den Destillationskolonnen K101 und K201 handelt es sich gemäß einer Ausführungsform der Erfindung um solche Kolonnen, die eine Auftrennung eines Kohlenwasserstoffstroms in C_{2/3}-Ströme, C₄-Ströme und C_{≥5}-Ströme erlauben. Die Kolonnen können als Trennwandkolonnen, Seitenkolonnen oder als 2-Kolonnen-Schaltungen ausgeführt sein. K301 ist gemäß einer Ausführungsform der Erfindung eine Kolonne, die die Trennung von Ethen und Propen erlaubt. Gemäß einer Ausführungsform der Erfindung ist der Reaktor R01 mit der Destillationskolonne K101 kombiniert zu einer Reaktivdestillationseinrichtung. Dabei erfolgt die Umsetzung direkt in der Destillationskolonne. Der Katalysator liegt in der Reaktionskolonne vor, so daß gleichzeitig mit der Umsetzung oder unmittelbar darauffolgend die Destillation ausgeführt wird. Ein entsprechendes Verfahren ist unter der Bezeichnung "Reaktivdestillation" bekannt.

Gemäß einer Ausführungsform sind Reaktor R02 und Destillationskolonne K201 zusammengefaßt zu einer Reaktivdestillationsvorrichtung, bei der Umsetzung und Destillation kombiniert sind entsprechend der vorstehend beschriebenen Reaktivdestillation.

Gemäß einer Ausführungsform der Erfindung finden beide Umsetzungsreaktionen in Reaktivdestillationsvorrichtungen statt. Bei beiden Umsetzungen handelt es sich um Gleichgewichtsreaktionen, so daß gemäß einer Ausführungsform der Erfindung die Verfahrensprodukte zur Erzielung eines möglichst hohen Umsatzes möglichst schnell aus dem Gleichgewicht entfernt werden. Dies ist insbesondere bei Verwendung von Reaktivdestillationsvorrichtungen möglich.

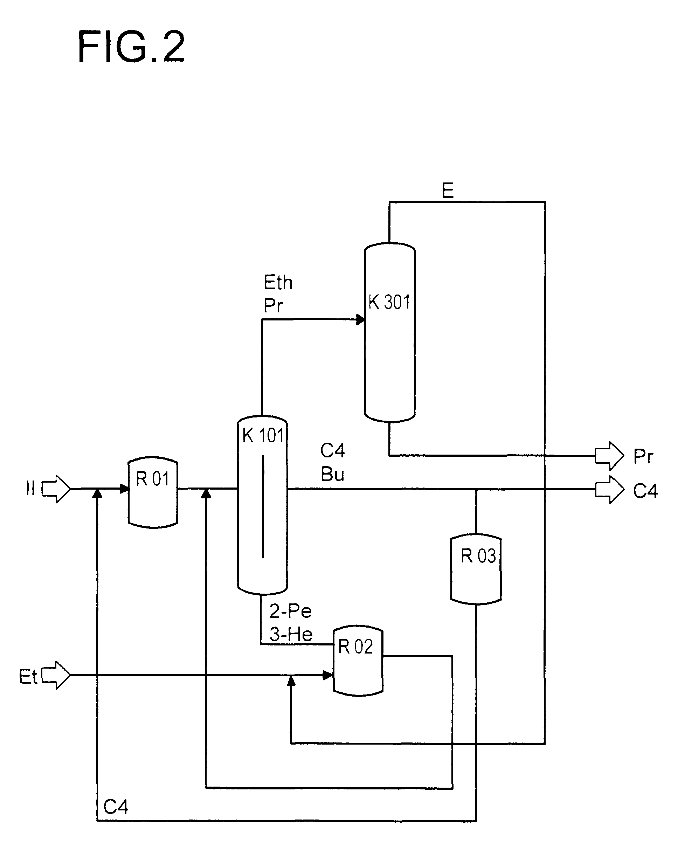

Eine weitere Ausführungsform des erfindungsgemäßen Verfahrens ist in Figur 2 dargestellt. Figur 2 zeigt ein Verfahren zur Herstellung von Propen durch
a) Umsetzung von 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
b) anschließendes Trennen des gebildeten Propens und 2-Pentens und der nicht umgesetzten Butene,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Überführen des umgesetzten Gemisches in Schritt b) zum Trennen des gebildeten Propens und 1-Butens,
e) zumindest teilweises Ausschleusen der in Schritt b) abgetrennten, nicht umgesetzten C₄-Fraktion und/oder zumindest teilweises Isomerisieren des an dieser C₄-Fraktion enthaltenen 1-Butens zu 2-Buten in Gegenwart eines Isomerisierungskatalysators und anschließendes Rückführen des erhaltenen Gemisches in Schritt a).

Für die Umsetzungsbedingungen gilt das vorstehend für das Verfahren gemäß Figur 1 Beschriebene analog. Das aus dem zweiten Metathesereaktor R02 erhaltene Gemisch wird direkt in die Destillationskolonne K101 zurückgeführt. Der in der Kolonne K101 erhaltene Mittelsiederaustrag aus C₄-Olefinen und Butanen wird zumindest teilweise ausgeschleust und/oder zumindest teilweise im Isomerisierungsreaktor R03 umgesetzt, wobei 1-Buten zu 2-Buten isomerisiert wird. Der Austrag aus dem Isomerisierungsreaktor R03 wird in Schritt a), d.h. den Metatheseraktor R01, zurückgeführt. Bei dieser Verfahrensvariante kann die Destillationskolonne K201 eingespart werden.

### METATHESE-KATALYSATOR

In den erfindungsgemäßen Verfahren können in R01 und R02 alle geeigneten Metathesekatalysatoren eingesetzt werden.

Gemäß einer Ausführungsform der Erfindung ist der Katalysator ein heterogener Katalysator, insbesondere ein Trägerkatalysator. Gemäß einer Ausführungsform der Erfindung enthält der Katalysator mindestens eine Verbindung eines Metalls der VIb, VIIb oder VIII Nebengruppe des Periodensystems der Elemente. Vorzugsweise enthält der Katalysator eine Rutheniumverbindung und/oder Rheniumverbindung. Derartige Katalysatoren sind beispielsweise beschrieben in K.J. Ivin, I.C. Mol, Olefin Metathesis and Metathesis Polymerization, 2nd Edition, Academic Press, New York, 1996; G.W. Parshall, S.D. Ittel, Homogeneous Catalysis, 2nd Edition, 1992, John Wiley & Sons, New York, Chichester, Brisbane, Toronto, Singapur, S. 217ff; R.H. Grubbs in Prog. Inorg. Chem., S. Lippard (Hrsg.), John Wiley & Sons, New York, 1978, Vol. 24, 1-50; R.H. Grubbs in Comprehensive Organomet. Chemie., G. Wilkinson (Hrsg.), Pergamon Press, Ltd., New York, 1982, Vol. 8, 499-551; D.S. Breslow, Prog. Polym. Sci. 1993, Vol. 18, 1141-1195.

Gemäß einer Ausführungsform der Erfindung handelt es sich bei der Metallverbindung um ein Metalloxid, Teiloxid mit zusätzlich vorliegenden organischen Resten oder um eine Carbonylverbindung.

Gemäß einer Ausführungsform der Erfindung wird ein homogener Katalysator eingesetzt. Der Katalysator ist dabei mindestens eine Verbindung eines Metalls der VIb, VIIb oder VIII Nebengruppe des Periodensystems der Elemente. Vorzugsweise wird Rhenium oder Ruthenium eingesetzt in den Metallverbindungen.

Vorzugsweise ist die Metallverbindung ein Oxid von Rhenium, insbesondere Re₂O₇.

### TRÄGER

Die erfindungsgemäßen Katalysatoren enthalten gemäß einer Ausführungsform der Erfindung einen Träger. Als Träger kommen dabei insbesondere anorganische Träger zur Anwendung, wie Al₂O₃, insbesondere γ-Al₂O₃, SiO₂, Fe₂O₃, oder deren Gemische, wie SiO₂/Al₂O₃, B₂O₃/SiO₂/Al₂O₃ oder Fe₂O₃/Al₂O₃.

Der Metalloxidgehalt auf dem Träger beträgt dabei gemäß einer Ausführungsform der Erfindung 1 bis 20 Gew.-%, vorzugsweise 3 bis 15 Gew.-%, insbesondere 8 bis 12 Gew.-%, bezogen auf das Gesamtgewicht des Trägerkatalysators.

Bevorzugt wird als Katalysator Re₂O₇ auf Al₂O₃, SiO₂/Al₂O₃, SiO₂/Al₂O₃/Fe₂O₃ oder B₂O₃/Al₂O₃ verwendet. Dabei beträgt der Anteil an Metalloxid vorzugsweise 1 bis 20 Gew.-%, besonders bevorzugt 3 bis 10 Gew.-%. Gemäß einer Ausführungsform der Erfindung wird MeReO₃ anstelle von Re₂O₇ oder im Gemisch damit verwendet.

Besonders bevorzugt wird erfindungsgemäß Re₂O₇ auf Al₂O₃ verwendet.

Die Katalysatoren werden gemäß einer Ausführungsform der Erfindung frisch calciniert eingesetzt, wobei sie keiner weiteren Aktivierung, beispielsweise durch Alkylierungsmittel bedürfen. Desaktivierte Katalysatoren können erfindungsgemäß durch Abbrennen von Coke-Rückständen beispielsweise bei 550°C im Luftstrom und Abkühlung unter Argon regeneriert werden.

Die erfindungsgemäßen Umsetzungen können dabei in Gegenwart eines Lösungsmittels, beispielsweise eines Kohlenwasserstofflösungsmittels durchgeführt werden. Gemäß einer bevorzugten Ausführungsform der Erfindung werden die Umsetzungen ohne weiteres zugesetztes Lösungsmittel durchgeführt.

### ISOMERISIERUNGS-KATALYSATOR

Als Isomerisierungs-Katalysator können alle Katalysatoren eingesetzt werden, die die Isomerisierung von 1-Buten zu 2-Buten katalysieren. Geeignete Isomerisierungskatalysatoren sind prinzipiell sämtliche homogenen oder heterogenen Edelmetallverbindungen, gegebenenfalls in Gegenwart von Wasserstoff, wie beispielsweise in A.J. Hubert, H. Reimbinger, Synthesis 1970,1,405 beschrieben.

Beispielsweise kann die Isomerisierung gemäß US 3,592,868 an RuO₃ auf einem anorganischen Träger wie SiO₂, Al₂O₃ oder TiO₂ oder gemischten Trägern erfolgen. In US 4,684,760 und US 4,889,840 sind Mischkatalysatoren, bestehend aus Magnesiumoxid, Zirkoniumoxid und einem Alkalimetalloxid auf einem anorganischen Träger beschrieben. In EP-A-0 129 899 und DE-A-34 27 979 sind geeignete Phosphorsäuren und Phosphate enthaltende Verbindungen sowie Zeolithe des Pentasil-Typs beziehungsweise übergangsmetall-dotierte Zeolithe beschrieben. Vorteilhaft hinsichtlich Katalysator- Standzeit und Reaktionsbedingungen sind die in US 5,177,281 beschriebenen Zeolithe des ZSM-Typs wie ZSM-22, ZSM-23 oder ZSM-35. Besonders aktive Palladium-Katalysatoren, beispielsweise auf Al₂O₃ als Träger, sind in US 3,531,545 beschrieben.

Der Isomerisierungskatalysator ist vorzugsweise ein heterogener Katalysator, der eine Verbindung eines Edelmetalls aus der Nebengruppe des Periodensystems der Elemente enthält. das in Form des Metalls oder eines Oxids oder Mischoxids vorliegen kann. Des weiteren sind Verbindungen eines Metalls der I. oder II. Hauptgruppe des Periodensystems der Elemente geeignet, die als Oxid oder Mischoxid vorliegen können.

Vorzugsweise wird ein Metall oder Metalloxid aus der VII. und VIII. Nebengruppe des Periodensystems der Elemente, das auf einem Träger vorliegen kann, in Inertgasatmosphäre oder in Gegenwart von Wasserstoff als Isomerisierungskatalysator eingesetzt.

Die bevorzugten erfindungsgemäßen Alkali- und/oder Erdalkalimetalloxidkatalysatoren werden vorzugsweise durch Tränkung von anorganischen Trägern wie SiO₂, Al₂O₃, ZrO₂, TiO₂ oder Gemischen davon mit Alkali- und/oder Erdalkaliverbindungen, nachfolgende Trocknung und Calzinieren zu den entsprechenden Oxiden hergestellt. Desaktivierter Katalysator kann durch Abbrennen von Coke-Rückständen bei Temperaturen oberhalb 350°C im Luftstrom und Abkühlung unter Inertgas-Atmosphere einfach regeneriert werden.

Besonders bevorzugt wird als Isomerisierungskatalysator PdO auf einem Al₂O₃- oder SiO₂-Träger in Gegenwart von Wasserstoff eingesetzt, wobei der Gehalt an Pd 0,1 bis 5 Gew.-%, bezogen auf das Gesamtgewicht des Katalysators. beträgt.

Beim Einsatz der vorstehenden Katalysatoren wird die C₄-Mittelsiederfraktion aus der Kolonne K201 nach partieller Isomerisierung in ein Gemisch aus 1-Buten und 2-Butenen überführt und zur Erhöhung der Propenausbeute in die Kreuzmetathese in R01 zurückgeführt. Alternativ kann 1-Buten bei entsprechender Reinheit des Raffinat II-Feedstroms ohne weitere Aufarbeitung gewonnen werden. Es kann dann beispielsweise zur Herstellung von Polymeren wie LLDPE-Copolymeren, HDPE-Copolymeren, Poly-1-buten oder zur Herstellung von Butylenoxid eingesetzt werden.

Bei der Isomerisierung werden - ebenso wie bei den Metathesereaktionen in R01 und R02 - die Bedingungen so gewählt, daß die Reaktionspartner in flüssiger Phase vorliegen. Die Temperatur beträgt so vorzugsweise 0 bis 200°C, besonders bevorzugt 50 bis 150°C. Der Druck beträgt vorzugsweise 2 bis 200 bar. Die Isomerisierung ist vorzugsweise nach einer Sekunde bis eine Stunde, vorzugsweise 5 bis 30 Minuten beendet. Sie kann kontinuierlich oder diskontinuierlich durchgeführt werden, wobei die Reaktoren wie die Metathesereaktoren Druckglas-Gefäße, Rohrreaktoren oder Destillationskolonnen sein können. Vorzugsweise werden auch hier Rohrreaktoren eingesetzt.

Die Erfindung betrifft auch eine Vorrichtung zur Durchführung der beschriebenen Verfahren. Eine Vorrichtung zur Durchführung des Verfahrens gemäß Figur 1 umfaßt einen Metathesereaktor (R01) zur Umsetzung von 1-Buten mit 2-Buten, an dessen Ausgang sich eine Destillationskolonne (K101) zur Trennung von C_{2/3}-Leichtsieder-, C₄-Mittelsieder- und C₅⁺-Hochsiederphasen anschließt, die als Trennwandkolonne ausgelegt sein kann, wobei der Leichtsiederausgang in eine Kolonne (K301) zur Trennung von Ethen und Propen führt, der Mittelsiederausgang zum Reaktor (R01) oder zur Ausschleusung führt und der Hochsiederausgang in einen Reaktor (R02) zur Umsetzung von 2-Penten mit Ethen, dessen Ausgang in die Kolonne (K101) führt, oder zur Ausschleusung führt, wobei der Ethenausgang aus der Kolonne (D3) und eine Ethenzuführung in den Reaktor (R2) führen.

Eine Vorrichtung zur Durchführung des Verfahrens gemäß Figur 1 umfaßt einen Methathesereaktor (R01) zur Umsetzung von 1-Buten mit 2-Buten, an dessen Ausgang sich eine Destillationskolonne (K101) zur Trennung von C_{2/3}-Leichtsieder-, C₄-Mittelsieder- und C₅⁺-Hochsiederphasen ausschließt, die als Trennwandkolonne, Seitenkolonne oder als 2-Kolonnen-Schaltung ausgelegt sein kann, wobei der Leichtsiederausgang in eine Kolonne (K301) zur Trennung von Ethen und Propen führt, der Mittelsiederausgang zum Reaktor (R01) oder zur Ausschleusung führt, und der Hochsiederausgang in einen Reaktor (R02) zur Umsetzung von 2-Penten mit Ethen führt, an dessen Ausgang sich eine Destillationskolonne (K201) zur Trennung von C_{2/3}-Leichtsieder-, C₄-Mittelsieder- und C₅⁺-Hochsiederphasen anschließt, die als Trennwandkolonne, Seitenkolonne oder als 2-Kolonnen-Schaltung ausgelegt sein kann, wobei der Leichtsiederausgang in die Kolonne (K301) führt, der Mittelsiederausgang in einen Isomerisierungsreaktor (R03) zur partiellen Isomerisierung von 1-Buten zu 2-Buten und gegebenenfalls zusätzlich zur Ausschleusung führt, wobei der Ausgang des Isomerisierungsreaktors (R03) gemeinsam mit dem Mittelsiederausgang von (K101) zum Reaktor (R01) führt und der Hochsiederausgang zur Ausschleusung führt, wobei der Ethenausgang aus der Kolonne (K301) und eine Ethenzuführung in den Reaktor (R02) führen.

### Beispiele

### Kontinuierliche Versuche zur Synthese von Propen aus Raffinat II

### Beispiel 1

### Kontinuierlicher Versuch zur Kreuzmetathese von 1-Buten mit 2-Buten Raffinat II)

Raffinat II (40 % 1-Buten, 45 % cis/trans-2Buten) wird bei 60°C, 25 bar und einer Katalysator-Belastung von 4500 kg/m² h kontinuierlich über einen mit Re₂O₇/Al₂O₃-Heterogenkontakt bestückten Rohrreaktor R01 geleitet. Der Reaktionsaustrag wird mittels Druckdestillation K101 (20 bar) in eine C2/3-Leichtsiederphase, eine C4-Olefine und Butane enthaltende Mittelsiederfraktion sowie eine aus 2-Penten und 3-Hexen bestehende Hochsiederfraktion getrennt. Die Prozent-Angaben beziehen sich auf Massenprozent:

| | **C2/3** | **C4** | **C5/6** |
|---|---|---|---|
| %m/m | 21 | 56 | 23 |

### Beispiel 2

### Kontinuierliche Versuche zur Ethenolyse des C5/C6-Hochsiederaustrages

Der aus 2-Penten und 3-Hexen bestehende Hochsiederaustrag aus K101 wird bei 60°C und 60 bar Ethen (Eduktverhältnis C2:C5,C6=1.1:1) bei einer mittleren Verweilzeit von 5 min kontinuierlich über einen mit Re₂O₇/Al₂O₃-Heterogenkontakt bestückten Rohrreaktor R02 geleitet. Der Reaktionsaustrag wird mittels Druckdestillation K201 (20 bar) in eine C2/3-Leichtsiederphase, eine 1-Buten enthaltende Mittelsiederfraktion sowie eine aus nicht umgesetzten 2-Penten und 3-Hexen bestehende Hochsiederfraktion getrennt. Die Prozent-Angaben beziehen sich auf Massenprozent:

| | **C2/3** | **1-Buten** | **C5/6** |
|---|---|---|---|
| %m/m | 41 | 52 | 7 |

### Beispiel 3

### Kontinuierliche Teilisomerisierung von 1-Buten

Der aus 1-Buten bestehende Mittelsiederaustrag aus Kolonne K201 wird bei 100°C und 15 bar bei einer mittleren Verweilzeit von 30 min in Gegenwart von 0,1 mol.-% Wasserstoff kontinuierlich über ein mit PdO/Al₂O₃-Heterogenkontakt bestücktes Strömungsrohr geleitet. Der Reaktionsaustrag wird gaschromatographisch analysiert und besteht zu 60% aus 1-Buten und 40% aus 2-Butenen.

## Patentansprüche

1. Verfahren zur Herstellung von Propen und gegebenenfalls 1-Buten durch
a) Umsetzung von 1-Buten und 2-Buten zu Propen und 2-Penten in Gegenwart eines Metathesekatalysators, der mindestens eine Verbindung eines Metalls oder VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) zumindest teilweises Ausschleusen des gebildeten 1-Butens und/oder zumindest teilweises Isomerisieren des gebildeten 1-Butens zu 2-Buten in Gegenwart eines Isomerisierungskatalysators und anschließendes Rückführen des nicht ausgeschleusten 1-Butens und gebildeten 2-Butens gemeinsam mit einem Teil der in Schritt a) nicht umgesetzten C₄-Fraktion in Schritt a).

2. Verfahren nach Anspruch 1, wobei das in Schritt c) gebildete 1-Buten teilweise in Gegenwart eines Isomerisierungskatalysators zu 2-Buten isomerisiert wird und das resultierende Gemisch aus 1-Buten und 2-Buten in Schritt a) zurückgeführt wird.

3. Verfahren nach Anspruch 1, wobei das in Schritt c) gebildete 1-Buten zumindest teilweise ausgeschleust wird und nicht ausgeschleustes 1-Buten in Schritt a) zurückgeführt wird.

4. Verfahren nach einem der Ansprüche, 1 bis 3, wobei Schritt b) eine Destillation ist, die in einer Trennwandkolonne, Seitenkolonne oder 2-Kolonnen-Schaltung durchgeführt werden kann, in der eine Propen enthaltende Leichtsiederphase, gegebenenfalls eine Butene enthaltende Mittelsiederphase und eine 2-Penten enthaltende Sumpfphase erhalten werden,
und/oder
wobei Schritt d) eine Destillation ist, die in einer Trennwandkolonne durchgeführt werden kann, in der eine Propen enthaltende Leichtsiederphase, eine 1-Buten enthaltende Mittelsiederphase und gegebenenfalls eine 2-Penten enthaltende Sumpfphase erhalten werden, wobei Schritte b) und d) in einer Destillationskolonne durchgeführt werden können.

5. Verfahren nach einem der Ansprüche 1 bis 4, wobei die Umsetzung in Schritten a) und/oder c) nicht vollständig erfolgt und in Schritt b) und/oder d) eine C_{2/3} enthaltende Leichtsiederphase, eine C₄-Mittelsiederphase und eine C_{≥5} enthaltende Sumpfphase erhalten werden,
wobei die gegebenenfalls zusammengefaßten Leichtsiederphasen durch Destillation in C₂- und C₃-Phasen getrennt werden und die C₂-Phase in Schritt c) zurückgeführt wird,
die gegebenenfalls zusammengefaßten Mittelsiederphasen zumindest teilweise in Schritt a) zurückgeführt werden und die gegebenenfalls zusammengefaßten Sumpfphasen zumindest teilweise in Schritt c) zurückgeführt werden.

6. Verfahren nach einem der Ansprüche 1 bis 5, wobei 1-Buten und 2-Buten als Gemisch eines C₄-Stroms, vorzugsweise aus einem Craker oder einer Raffinerie, insbesondere als Raffinat II eingesetzt werden, wobei der C₄-Strom vor der Umsetzung zur Reinigung über Absorbermaterialien geleitet werden kann.

7. Verfahren nach Anspruch 6, wobei das Verhältnis von Molanteil 1-Buten zu Molanteil 2-Buten 10:1 bis 1:10, beträgt.

8. Verfahren nach einem der Ansprüche 1 bis 7, wobei ein heterogener Metathesakatalysator eingesetzt wird, der eine Rhenium- oder Rutheniumverbindung enthält, vorzugsweise ein Rheniumoxid auf einem anorganischen Träger.

9. Verfahren nach Anspruch 8, wobei der Metathesekatalysator Re₂O₇ auf einem Al₂O₃-Träger enthält oder daraus besteht, wobei der Rheniumoxidgehalt 1 bis 20 Gew.-%, vorzugsweise 3 bis 10 Gew.-%, bezogen auf das Gesamtgewicht des Katalysators, beträgt.

10. Verfahren nach einem der Ansprüche 1 bis 9, wobei die Umsetzung von 1-Buten und 2-Buten und/oder die Umsetzung von 2-Penten mit Ethen als Reaktivdestillation durchgeführt werden.

11. Verfahren nach einem der Ansprüche 1 bis 10, wobei als Isomerisierungskatalysator eine Edelmetallverbindung oder dessen Oxid, das auf einem Träger vorliegen kann, in Gegenwart einer Inertgas- oder Wasserstoffatmosphäre eingesetzt wird.

12. Verfahren nach einem der Ansprüche 1 bis 11, **dadurch gekennzeichnet, daß** zwischen den Schritten b) und c) der abgetrennte, 2-Penten und 3-Hexen enthaltende Hochsiederaustrag einer Destillation zur Trennung von 2-Penten und 3-Hexen unterworfen wird.

13. Verfahren zur Herstellung von Propen durch
a) Umsetzung von 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
b) anschließendes Trennen des gebildeten Propens und 2-Pentens und der nicht umgesetzten Butene,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Überführen des umgesetzten Gemisches in Schritt b) zum Trennen des gebildeten Propens und 1-Butens,
e) zumindest teilweises Ausschleusen der in Schritt b) abgetrennten, nicht umgesetzten C₄-Fraktion und/oder zumindest teilweises Isomerisieren des in dieser C₄-Fraktion enthaltenen 1-Butens zu 2-Buten in Gegenwart eines Isomerisierungskatalysators und anschließendes Rückführen des erhaltenen Gemisches in Schritt a).

14. Vorrichtung zur Durchführung eines Verfahrens nach einem der Ansprüche 1 bis 11, umfassend einen Methathesereaktor (R01) zur Umsetzung von 1-Buten mit 2-Buten, an dessen Ausgang sich eine Destillationskolonne (K101) zur Trennung von C_{2/3}-Leichtsieder-, C₄-Mittelsieder- und C₅⁺-Hochsiederphasen ausschließt, die als Trennwandkolonne, Seitenkolonne oder 2-Kolonnen-Schaltung ausgelegt sein kann, wobei der Leichtsiederausgang in eine Kolonne (K301) zur Trennung von Ethen und Propen führt, der Mittelsiederausgang zum Reaktor (R01) oder zur Ausschleusung führt, und der Hochsiederausgang in einen Reaktor (R02) zur Umsetzung von 2-Penten mit Ethen führt, an dessen Ausgang sich eine Destillationskolonne (K201) zur Trennung von C_{2/3}-Leichtsieder-, C₄-Mittelsieder- und C₅⁺-Hochsiederphasen anschließt, die als Trennwandkolonne, Seitenkolonne oder 2-Kolonnen-Schaltung. ausgelegt sein kann, wobei der Leichtsiederausgang in die Kolonne (K301) führt, der Mittelsiederausgang in einen Isomerisierungsreaktor (R03) zur partiellen Isomerisierung von 1-Buten zu 2-Buten und gegebenenfalls zusätzlich zur Ausschleusung führt, wobei der Ausgang des Isomerisierungsreaktors (R03) gemeinsam mit dem Mittelsiederausgang von (K101) zum Reaktor (201) führt und der Hochsiederausgang zur Rückführung in (R02) oder zur Ausschleusung führt, wobei der Ethenausgang aus der Kolonne (K301) und eine Ethenzuführung in den Reaktor (R02) führen.

## Claims

1. A process for preparing propene and, if desired, 1-butene by
a) reacting 1-butene and 2-butene to give propene and 2-pentene in the presence of a metathesis catalyst comprising at least one compound of a metal of transition group VIb, VIIb or VIII of the Periodic Table of the Elements,
b) subsequently separating the propene and 2-pentene formed,
c) subsequently reacting the 2-pentene with ethene to give propene and 1-butene in the presence of a metathesis catalyst comprising at least one compound of a metal of transition group VIb, VIIb or VIII of the Periodic Table of the Elements,
d) subsequently separating the propene and 1-butene formed,
e) discharging at least some of the 1-butene formed and/or at least partly isomerizing the 1-butene formed to give 2-butene in the presence of an isomerization catalyst and subsequently returning the undischarged 1-butene and the 2-butene formed together with a part of the C₄ fraction not reacted in step a) to step a).

2. A process as claimed in claim 1, wherein the 1-butene formed in step c) is at least partially isomerized in the presence of an isomerization catalyst to give 2-butene and the resulting mixture of 1-butene and 2-butene is returned to step a).

3. A process as claimed in claim 1, wherein at least some of the 1-butene formed in step c) is discharged and undischarged 1-butene is returned to step a).

4. A process as claimed in any of claims 1 to 3, wherein step b) is a distillation which can be carried out in a dividing wall column, a side column or a 2-column arrangement in which a propene-containing low-boiling phase, possibly a butene-containing intermediate-boiling phase and a 2-pentene-containing bottom phase are obtained,
and/or
wherein step d) is a distillation which can be carried out in a dividing wall column in which a propene-containing low-boiling phase, a 1-butene-containing intermediate-boiling phase and possibly a 2-pentene-containing bottom phase are obtained, wherein steps b) and d) can be carried out in a distillation column.

5. A process as claimed in any of claims 1 to 4, wherein the reaction in steps a) and/or c) is not carried to completion and in step b) and/or d) a C₂/₃-containing low-boiling phase, a C₄ intermediate-boiling phase and a C_{≥5}-containing bottom phase are obtained,
wherein the low-boiling phases, which may be combined, are separated by distillation into C₂ and C₃ phases and the C₂ phase is returned to step c),
at least some of the intermediate-boiling phases, which may be combined, are returned to step a) and at least some of the bottom phases, which may be combined, are returned to step c).

6. A process as claimed in any of claims 1 to 5, wherein 1-butene and 2-butene are used as a mixture in a C₄ stream, preferably from a cracker or a refinery, in particular as raffinate II, wherein the C₄ stream can be passed over absorber materials before the reaction to purify it.

7. A process as claimed in claim 6, wherein the molar ratio of 1-butene to 2-butene is from 10:1 to 1:10.

8. A process as claimed in any of claims 1 to 7, wherein use is made of a heterogeneous metathesis catalyst comprising a rhenium or ruthenium compound, preferably a rhenium oxide, on an inorganic support.

9. A process as claimed in claim 8, wherein the metathesis catalyst comprises or consists of Re₂O₇ on an Al₂O₃ support, with the rhenium oxide content being from 1 to 20 % by weight, preferably from 3 to 10 % by weight, based on the total weight of the catalyst.

10. A process as claimed in any of claims 1 to 9, wherein the reaction of 1-butene and 2-butene and/or the reaction of 2-pentene with ethene are carried out as a reactive distillation.

11. A process as claimed in any of claims 1 to 10, wherein the isomerization catalyst used is a noble metal compound or its oxide, which may be present on a support, in the presence of an inert gas or hydrogen atmosphere.

12. A process as claimed in any of claims 1 to 11, wherein, between steps b) and c), the high-boiling product comprising 2-pentene and 3-hexene which has been separated off is subjected to a distillation to separate 2-pentene and 3-hexene.

13. A process for preparing propene by
a) reacting 1-butene and 2-butene to give propene and 2-pentene in the presence of a metathesis catalyst comprising at least one compound of a metal of transition group VIb, VIIb or VIII of the Periodic Table of the Elements,
b) subsequently separating the propene and 2-pentene formed and the unreacted butenes,
c) subsequently reacting the 2-pentene with ethene to form propene and 1-butene in the presence of a metathesis catalyst comprising at least one compound of a metal of transition group VIb, VIIb or VIII of the Periodic Table of the Elements,
d) subsequently transferring the unreacted mixture to step b) to separate the propene and 1-butene formed,
e) discharging at least some of the unreacted C₄ fraction separated off in step b) and/or at least partly isomerizing the 1-butene present in this C₄ fraction to give 2-butene in the presence of an isomerization catalyst and subsequently returning the mixture obtained to step a).

14. An apparatus for carrying out a process as claimed in any of claims 1 to 11, comprising a metathesis reactor (R01) for reacting 1-butene with 2-butene whose outlet leads to a distillation column (K101), which can be configured as a dividing wall column, a side column or a 2-column arrangement, for separating C_{2/3} low-boiling, C, intermediate-boiling and C₅⁺ high-boiling phases, where the low boiler outlet leads to a column (K301) for separating ethene and propene, the intermediate boiler outlet leads to the reactor (R01) or to discharge and the high boiler outlet leads to a reactor (R02) for reacting 2-pentene with ethene whose outlet leads to a distillation column (K201), which can be configured as a dividing wall column, a side column or a 2-column arrangement, for separating C_{2/3} low-boiling, C₄ intermediate-boiling and C₅⁺ high-boiling phases, where the low boiler outlet leads to the column (K301), the intermediate boiler outlet leads to an isomerization reactor (R03) for partially isomerizing 1-butene to 2-butene and, if desired, additionally to discharge, where the outlet of the isomerization reactor (R03) together with the intermediate boiler outlet from (K101) leads to the reactor (R01) and the high boiler outlet leads to the return to (R02) or to discharge, where the ethene outlet from the column (K301) and an ethene feed line lead to the reactor (R02).

## Revendications

1. Procédé pour la préparation du propène et éventuellement du 1-butène comprenant les étapes consistant à :
a) faire réagir du 1-butène et du 2-butène en propène et en 2-pentène en présence d'un catalyseur de métathèse comprenant au moins un composé d'un métal ou d'un élément du Groupe Secondaire VIb, VIIb ou VIII de la Classification Périodique des Eléments,
b) puis à séparer le 2-pentène et le propène formés,
c) puis à faire réagir le 2-pentène avec l'éthène en propène et en 1-butène, en présence d'un catalyseur de métathèse qui comprend au moins un composé d'un métal du Groupe Secondaire VIb, VIIb ou VIII de la Classification Périodique des Eléments,
d) puis à séparer le 1-butène et le propène formés,
e) éliminer, au moins partiellement, le 1-butène formé et/ou isomériser, au moins partiellement, le 1-butène formé en 2-butène, en présence d'un catalyseur d'isomérisation, puis à recycler conjointement, dans l'étape a), le 1-butène qui n'a pas été éliminé, le 2-butène formé et une partie de la fraction en C₄ qui, dans l'étape a), n'a pas réagi.

2. Procédé selon la revendication 1, dans lequel l'on procède à une isomérisation partielle du 1-butène formé dans l'étape c) en 2-butène, en présence d'un catalyseur d'isomérisation, puis l'on recycle dans l'étape a) le mélange résultant constitué de 1-butène et de 2-butène.

3. Procédé selon la revendication 1, dans lequel l'on élimine, au moins partiellement, le 1-butène formé dans l'étape c) et on recycle dans l'étape a) le 1-butène que l'on n'a pas éliminé.

4. Procédé selon l'une quelconque des revendications 1 à 3, dans lequel l'étape b) est une distillation que l'on peut réaliser dans une colonne à cloison de séparation, dans une colonne latérale ou dans deux colonnes couplées, et dans laquelle on obtient une phase de composés à bas point d'ébullition contenant le propène, éventuellement une phase de composés à point d'ébullition moyen contenant le butène et une phase de fond contenant le 2-pentène, et/ou
dans lequel l'étape d) est une distillation que l'on peut réaliser dans une colonne à cloison de séparation, dans laquelle on obtient une phase de composés à bas point d'ébullition contenant le propène, une phase de composés à point d'ébullition moyen contenant le 1-butène et éventuellement une phase de fond contenant le 2-pentène, procédé dans lequel on peut réaliser les étapes b) et d) dans une colonne de distillation.

5. Procédé selon une quelconque des revendications 1 à 4, dans lequel l'on procède à une réaction incomplète dans les étapes a) et/ou c), et dans lequel on obtient dans l'étape b) et/ou c) une phase de composés à bas point d'ébullition contenant des composés en C₂ à C₃, une phase de composés en C₄ à point d'ébullition moyen et une phase de fond contenant des composés en C_{≥5},
dans lequel l'on sépare par distillation les phases de composés à bas point d'ébullition que l'on a éventuellement réunies auparavant, pour obtenir des phases de composés en C₂ et de composés en C₃, puis l'on recycle dans l'étape c) la phase en composés en C₂,
l'on recycle dans l'étape a), au moins partiellement, les phases de composés à point d'ébullition moyen que l'on a éventuellement réunies auparavant, et on recycle dans l'étape c), au moins partiellement, les phases de fond que l'on a éventuellement réunies auparavant.

6. Procédé selon l'une quelconque des revendications 1 à 5, dans lequel l'on met en oeuvre le 1-butène et le 2-butène en tant que mélange d'un courant en C₄, de préférence provenant d'un craqueur ou d'une raffinerie, en particulier en tant que produit raffiné II, mise en oeuvre lors de laquelle on conduit éventuellement le courant en C₄ sur des matières absorbantes afin de le purifier, avant la réaction.

7. Procédé selon la revendication 6, dans lequel le rapport molaire entre le 1-butène et le 2-butène est compris entre 10:1 et 1:10.

8. Procédé selon l'une quelconque des revendications 1 à 7, dans lequel on met en oeuvre un catalyseur de métathèse hétérogène qui contient un composant du rhénium ou du ruthénium, de préférence l'oxyde de rhénium sur un support inorganique.

9. Procédé selon la revendication 8, dans lequel le catalyseur de métathèse contient le Re₂O₇ sur un support constitué de Al₂O₃ ou bien en est constitué, la teneur en oxyde de rhénium étant comprise entre 1% et 20% en poids, de préférence entre 3% et 10% en poids, par rapport au poids total du catalyseur.

10. Procédé selon l'une quelconque des revendications 1 à 9, dans lequel on réalise la réaction du 1-butène et du 2-butène et/ou la réaction du 2-pentène avec l'éthène sous la forme d'une distillation réactive.

11. Procédé selon l'une quelconque des revendications 1 à 10, dans lequel on met en oeuvre, en tant que catalyseur d'isomérisation, un composé de métal noble ou son oxyde qui est éventuellement présent sur un support, en présence d'une atmosphère de gaz inerte ou d'hydrogène.

12. Procédé selon l'une quelconque des revendications 1 à 11, **caractérisé en ce que** l'on soumet à une distillation le courant de composés à haut point d'ébullition contenant le 2-pentène et le 3-hexène, que l'on a séparé entre les étapes b) et c), afin de séparer le 2-pentène et le 3-hexène.

13. Procédé pour la préparation du propène comprenant les étapes consistant à
a) faire réagir du 1-butène et du 2-butène en propène et en 2-pentène, en présence d'un catalyseur de métathèse comprenant au moins un composé d'un métal du Groupe Secondaire VIb, VIIb ou VIII de la Classification Périodique des Eléments,
b) puis à séparer le 2-pentène et le propène formés et du butène n'ayant pas réagi,
c) puis à faire réagir le 2-pentène avec l'éthène en propène et en 1-butène, en présence d'un catalyseur de métathèse qui comprend au moins un composé d'un métal du Groupe Secondaire VIb, VIIb ou VIII de la Classification Périodique des Eléments,
d) puis à conduire le mélange ayant réagi dans l'étape b) vers l'étape de séparation du 1-butène et du propène formés,
e) éliminer, au moins partiellement, la fraction en C₄ qui a été séparé dans l'étape b) et qui n'a pas réagi et/ou une isomérisation, au moins partielle, du 1-butène qui est contenu dans cette fraction en C₄ afin d'obtenir le 2-butène, en présence d'un catalyseur d'isomérisation, suivi d'un recyclage dans l'étape a) du mélange obtenu.

14. Dispositif pour la réalisation du procédé selon l'une quelconque des revendications 1 à 11, comprenant un réacteur de métathèse (R01) pour la réaction du 1-butène avec le 2-butène, présentant à sa sortie une colonne de distillation (K101) destinée à la séparation des composés en C₂ à C₃ à bas point d'ébullition, des composés en C₄ à point d'ébullition moyen et des composés en C_{≥5} à haut point d'ébullition, qui peut être sous la forme d'une colonne à cloison de séparation, d'une colonne latérale ou de deux colonnes couplées, dans laquelle la sortie pour les composés à bas point d'ébullition conduit dans une colonne (K301) destinée à la séparation de l'éthène et du propène, la sortie pour les composés à point d'ébullition moyen conduit dans le réacteur (R01) ou à l'élimination, et la sortie pour les composés à haut point d'ébullition conduit dans un réacteur (R02) destiné à la réaction du 2-pentène avec l'éthène, qui présente à sa sortie une colonne de distillation (K201) destinée à la séparation des composés en C₂ à C₃ à bas point d'ébullition, des composés en C₄ à point d'ébullition moyen et des composés en C_{≥5} à haut point d'ébullition, et qui peut être présent sous forme de colonne à cloison de séparation, de colonne latérale ou de couplage de deux colonnes, dans laquelle la sortie pour les composés à bas point d'ébullition conduit dans la colonne (K301), la sortie pour les composés à point d'ébullition moyen conduit dans un réacteur d'isomérisation (R03) destiné à l'isomérisation partielle du 1-butène en 2-butène et, éventuellement, conduit de plus à l'élimination, où la sortie du réacteur d'isomérisation (R03) et la sortie du (K101) des composés à point d'ébullition moyen conduisent conjointement au réacteur (201), et la sortie pour les composés à haut point d'ébullition conduit au recyclage dans (R02) ou bien à l'élimination, la sortie pour l'éthène de la colonne (K301) et un dispositif d'alimentation en éthène conduisant dans le réacteur (R02).
